# EUROPÄISCHE PATENTSCHRIFT

(11) **EP 0 835 062 B1**
(45) Veröffentlichungstag und Bekanntmachung des Hinweises auf die Patenterteilung: **21.08.2002**
(21) Anmeldenummer: 97918094.0
(22) Anmeldetag: 01.04.1997
(51) Int. Cl.: A23J 1/00, A23J 1/10, C07K 1/12

(54) **VERFAHREN ZUR ERZEUGUNG VON PROTEIN-HYDROLISATEN**
PROCESS FOR PRODUCING PROTEIN HYDROLYSATES
PROCEDE DE PRODUCTION D'HYDROLYSATS PROTEIQUES

(30) Priorität: 28.03.1996 DE 19612281
(43) Veröffentlichungstag der Anmeldung: 15.04.1998
(73) Patentinhaber: GUR Gesellschaft für Umwelttechnik und Recycling Mbh, 83043 Bad Aibling (DE)
(72) Erfinder: BREHM, Josef, D-83555 Gars (DE); BERNDT, Claus, D-83043 Bad Aibling (DE); STENGELE, Klaus-Peter, D-84568 Pleinskirchen (DE); KOLBECK, Winfried, D-81247 München (DE)
(74) Vertreter: Thiel, Christian
(86) Internationale Anmeldenummer: EP9701642
(87) Internationale Veröffentlichungsnummer: WO97036500

(56) Entgegenhaltungen:
- DE-A- 3 713 179
- DE-A- 4 231 914
- DATABASE WPI Section Ch, Week 9241 Derwent Publications Ltd., London, GB; Class D12, AN 92-339155 XP002037599 & SU 1 692 503 A (AZOV BLACK SEA FISHING OCEANOGRAPHY INST) , 23.November 1991

## Beschreibung

Die Erfindung betrifft ein Verfahren zur Erzeugung von Proteinhydrolysaten aus animalischen und/oder vegetabilen Ausgangsmaterialien durch Zerkleinern und Anmaischen der zerkleinerten Ausgangsmaterialien zu einer pumpfähigen Masse, Aufschließen der angemaischten Ausgangsmaterialien in einer elektrischen Entladungsstrecke und Abtrennen der wäßrigen Phase des Aufschlusses von festen und nicht wassermischbaren Rückständen.

Proteinhaltige Rückstände der industriellen oder handwerklichen Verwertung landwirtschaftlicher Produkte wie auch pflanzliche Rohstoffe stellen wegen der darin enthaltenen Inhaltsstoffe wertvolle Rohprodukte dar. Ein Problem ist aber häufig die kostengünstige und einfache Gewinnung dieser Inhaltsstoffe. Insbesondere animalische Schlachtabfälle, wie Häute, Haare, Borsten, Federn, Knochen, Fleischteile, Blut, etc. entziehen sich weitgehend einer einfachen und kostengünstigen weiteren Verarbeitung, was ihre Inhaltsstoffe angeht. Dabei stellen die darin enthaltenen Proteine eine wertvolle Rohstoffquelle dar, die bislang nur zu einem geringen Teil genutzt wird.

Das erfindungsgemäße Verfahren entwickelt eine Technik weiter, die aus der DE 42 31 914 A1 bekannt ist. Dort ist ein Verfahren zur Erzeugung von Proteinhydrolisaten aus animalischen und/oder vegetabilen Substraten durch Zerkleinern der Substrate, Erhitzen des zerkleinerten Substrats, Anmaischen des erhitzten Substrats zu einer pumpfähigen Masse, Aufschließen des angemaischten Substrats mittels elektrischer Entladungen und Abtrennen der wäßrigen Phase des Aufschlusses von festen und nicht wassermischbaren flüssigen Rückständen beschrieben, bei dem die Temperatur während des gesamten Verfahrens unter der Koagulationstemperatur von Eiweiß gehalten wird und die Einmaischung mit aus dem Aufschluß erhaltener wäßriger Hydrolisatphase vorgenommen wird. Dieses Verfahren hat durchaus seine Vorteile, ist jedoch hinsichtlich des Energieeinsatzes und der Aufarbeitung der dabei anfallenden Produkte noch verbesserungsfähig. Insbesondere ist die Abtrennung der erhaltenen Proteinhydrolisate von den festen und nicht wassermischbaren flüssigen Rückständen aufwendig und kostenintensiv. Zwar kann die Abtrennung und anschließende Filtration der erhaltenen Hydrolisatlösung durch eine entsprechende Vorbehandlung der Ausgangsmaterialien verbessert werden, jedoch stellt dies einen zusätzlichen Schritt mit zusätzlichen Kosten dar.

Aus der DE-A-37 13 179 A1 ist ferner ein Verfahren zur Gewinnung von Gelatine und diese enthaltenden Produkten bekannt, bei dem kollagenhaltiges Ausgangsmaterial in einem wäßrigen Medium mit Hilfe von elektrischem Strom aufgeschlossen wird. Als kollagenhaltiges Ausgangsmaterial kommen dabei animalische Schlachtabgänge zum Einsatz. Der Aufschluß erfolgt mit einem elektrischen Entladungsstrom mit kurzzeitigen Entladungsimpulsen. Erhalten wird bei dem Verfahren ein kollagenhaltiges Material, das insbesondere als Leim Verwendung findet.

Die in diesem bekannten Verfahren zum Einsatz kommende Entladungsvorrichtung ist an und für sich bekannt und wurde für eine Reihe von Einsatzzwecken beschrieben, darunter den oben angegebenen. Zur Entladungstechnik wird im übrigen auf die DE-PS-12 37 541 und 19 46 267 sowie auf DE-OS-16 67 029, 17 92 572, 29 07 887 und DE 31 16 623 A1 wie auch auf die Angaben in DE 37 13 179 A1 verwiesen.

Schließlich geht aus der DE-OS 27 50 149 ein Verfahren zur Herstellung keratinartigen Proteins hervor, bei dem Keratin mit Sattdampf unter Druck hydrolysiert wird. Die Verfahrensbedingungen sind recht drastisch und führen zu hohen Kosten und einem chemisch veränderten Produkt.

Ziel der Erfindung ist ein Verfahren, mit dem die in animalischen und vegetabilen Rohstoffen, insbesondere Schlachtabfällen und pflanzlichen landwirtschaftlichen Rohprodukten und Abfällen enthaltenen Proteine einfach, kostengünstig und weitgehend rein abgetrennt werden können.

Dieses Ziel wird mit einem Verfahren der eingangs beschriebenen Art erreicht, bei dem das Ausgangsmaterial bei einem pH-Wert zwischen 10,0 und 14,0 in der Entladungsstrecke aufgeschlossen wird und das aufgeschlossene Material einer Nachreaktion über wenigstens 1 h bei einem pH-Wert von 10,0 bis 14,0 unterworfen wird, wobei die Temperatur wenigstens 70°C beträgt.

Als animalische Rohstoffe kommen Häute, Haare, Borsten, Federn, Knochen, Hornbestandteile, Fleischteile, Blut und dergleichen Schlachtnebenprodukte in Frage. Als vegetabile Substrate können beliebige proteinhaltige pflanzliche Produkte eingesetzt werden, insbesondere Abfallstoffe der Landwirtschaft und der landwirtschaftliche Produkte verarbeitenden Industrie.

Die zum Einsatz kommenden Substrate werden zu Beginn des Verfahrens zerkleinert, beispielsweise mit üblichen Mühlen, Brechern, Wölfen und dergleichen. Dabei sollte eine möglichst feine Zerkleinerung erreicht werden, da dies den späteren Aufschluß der Substrate erleichtert und ihre Pumpfähigkeit fördert. Hierzu sollte die Partikelgröße ¾ 6 - 8 mm sein. Dabei kann das Substrat beispielsweise unter Zuhilfenahme von flüssigem Stickstoff gekühlt und dann mechanisch zermahlen werden.

Die zerkleinerten Substrate können im nächsten Schritt auf eine Temperatur unterhalb der Koagulationstemperatur von Eiweiß erhitzt werden, vorzugsweise auf eine Temperatur unter etwa 70°C. Dies kann sich bei fettreichen Ausgangsmaterialien als vorteilhaft erweisen, da hierbei eine Abscheidung vorhandener Fettbestandteile erfolgt, die aus dem Verfahren durch Abschöpfen, Ablaufenlassen oder Dekantieren entfernt werden. Bei geringen oder normalen Fettanteilen ist dieser Schritt aber in der Regel nicht erforderlich.

Anschließend wird das Substrat zu einer pumpfähigen Masse angemaischt, wozu Wasser oder, wenn hohe Produktkonzentrationen erwünscht sind, aus dem Aufschluß erhaltene wäßrige Hydrolysatphase verwandt wird. In der Praxis hat sich das Anmaischen mit Wasser als zweckmäßig erwiesen.

Dem angemaischten Substrat wird dann eine Base zugesetzt, die den pH-Wert auf den gewünschten Wert von pH 10,0 bis 14,0 und die Stromleitfähigkeit der Masse erhöht. Weiterhin können Elektrolyte zugesetzt werden, falls darüber hinaus erforderlich. Diese Elektrolyte sollen mit dem Substrat und mit den Produkten keine chemischen Verbindungen eingehen und aus der Mischung mit einfachen Verfahren abtrennbar sein. Beispielsweise können hierzu in wäßriger Phase lösliche anorganische Salze oder organische Verbindungen eingesetzt werden, beispielsweise Kochsalz oder Zimtsäure. Eine ausreichende Stromleitfähigkeit ist aber in der Regel durch die Base gegeben. Vorzugsweise wird Natronlauge eingesetzt. Kalkmilch kann verwandt werden, sofern dies das ausgewählte Aufbereitungsverfahren (Filtration) zuläßt.

Das angemaischte Substrat wird anschließend durch Einwirkung elektrischer Impulse in einer Entladungsstrecke aufgeschlossen. Durch die elektrischen Impulse werden die Zellen aufgebrochen und darin enthaltenes Protein freigesetzt und in die wäßrige Phase überführt. Die Proteine selbst sind als amphotere Stoffe polarisierbar und werden unter Einfluß der Stromimpulse hydrolytisch gespalten, so daß sie - ggf. erst nach mehrmaligem Durchgang - in ein Peptongemisch bis hin zu einzelnen Aminosäuren aufgespalten sind. Auf diese Weise wird aus dem eingesetzten Substrat im wesentlichen reines Protein herausgelöst, während die verbleibenden Bestandteile im wesentlichen in fester oder suspendierter Form oder als Fettphase zurückbleiben.

Beim Durchgang durch die Entladungsstrecke heizen sich die angemaischten Ausgangsmaterialien auf. Dabei sollte die Temperatur unterhalb 95°C gehalten werden, insbesondere bei einer Endtemperatur im Bereich von 70 bis 85°C. Die Temperatur kann dabei in wirkungsvoller Weise durch die Fließgeschwindigkeit geregelt werden, die im allgemeinen auf Werte zwischen 1,5 und 5 l/min., vorzugsweise 2 bis 4 l/min. eingestellt wird. Hohe Durchlaufgeschwindigkeiten können es erforderlich machen, die Zahl der Durchgänge durch die Entladungsstrecke zu erhöhen, also das angemaischte Ausgangsmaterial im Kreislauf zu führen.

Während des Aufschlusses liegt der pH-Wert des Materials zweckmäßigerweise im Bereich von 11,0 bis 13,5, wobei zu berücksichtigen ist, daß durch den Aufschluß selbst im allgemeinen eine Erniedrigung des pH-Wertes eintritt. Der pH-Wert sollte so hoch gewählt werden, daß er auch am Ende des Aufschlusses noch innerhalb des Bereiches liegt.

An die elektrische Entladungsstrecke und den darin bewirkten Aufschluß schließt sich eine Nachreaktion an, die bei einem pH-Wert von 10,0 bis 14,0 über einen Zeitraum von wenigstens 1 h und einer Temperatur von wenigstens 70°C durchgeführt wird. Zweckmäßigerweise liegt der pH-Wert im Bereich von 11,0 bis 13,5, wobei die Temperatur bis auf einen Wert von 95°C gesteigert werden kann. Die Nachreaktionszeit beträgt vorzugsweise 2 h bis 15 h. In der Praxis hat es sich als vorteilhaft erwiesen, die Nachreaktion über Nacht durchzuführen.

Durch die Nachreaktion wird das in der Entladungsstrecke behandelte Material weiter aufgeschlossen. Insbesondere ergibt sich durch die Kombination von basischem pH-Wert und hoher Temperatur eine weitere Abtrennung vorhandener Fettbestandteile, die im Anschluß an die Nachreaktionszeit als an der Oberfläche schwimmende Fettphase dekantiert oder abgepumpt werden kann. Feste Bestandteile sedimentieren, was dem anschließenden Trennprozeß förderlich ist.

Im Anschluß an die Nachreaktion wird das aufgeschlossene Substrat einer Trennstrecke zugeführt, in der zunächst die festen Bestandteile abgepreßt werden und anschließend die wäßrige Phase gesammelt wird.

Die wäßrige Phase wird vor der weiteren Behandlung neutralisiert, wobei der pH-Wert vorzugsweise auf einen Wert von 5,5 bis 7,0, insbesondere etwa 6,5, eingestellt wird. Zur Neutralisation können beliebige Säuren eingesetzt werden, jedoch ist die Verwendung von Schwefelsäure bevorzugt.

Das neutralisierte Produkt wird, vorzugsweise mit einer Temperatur von 50 bis 80°C, insbesondere 65 bis 75°C, filtriert, wobei zur Erzielung eines sterilen Produkts eine Mikrofiltration bevorzugt ist. Im Anschluß an die Filtration wird das Produkt dann entwässert, beispielsweise durch Verdampfen der wäßrigen Phase bei Normal- oder vermindertem Druck, durch Gefriertrocknen oder Sprühtrocknung.

Der Aufschluß des angemaischten und ggf. mit einem Elektrolyten versetzten Substrats erfolgt vorzugsweise im Elektroimpulsverfahren in einer an und für sich bekannten Entladungsstrecke. Hierbei wird über einen Generator eine Hochspannung erzeugt. Mittels Hochleistungskondensatoren werden Entladungen im Mikrosekundenbereich erzeugt, beispielsweise 5 bis 30 Entladungen pro Sekunde, die über an der Entladungsstrecke paarweise angeordnete Elektroden auf das Substratgemisch gegeben werden. Die Entladungen selbst haben eine Dauer von Mikrosekunden. Zur Technik wird auf die eingangs genannten Druckschriften hingewiesen.

Beim Aufschluß werden im Substrat eingelagertes Eiweiß und darin enthaltene Kollagene von der Zellstruktur gelöst, treten aus der Zelle aus und gehen in wäßrige Lösung über. Die Impulsstöße der Entladungsstrecke werden so gesteuert, daß ein Verbrennen oder eine unzulässige Temperaturerhöhung nicht eintreten. Hierzu ist eine Temperaturüberwachung von Vorteil. Für den Fall einer unzulässigen Temperatursteigerung kann die Impulszahl zurückgenommen, die Durchlaufgeschwindigkeit der Maische erhöht oder die die Entladungsstrecke passierende Maische gekühlt werden, beispielsweise über eine im Bereich der Entladungsstrecke an- oder nachgeordnete Kühlung oder separate Kühlstrecke. Vorzugsweise wird die Temperatur des angemaischten Ausgangsmaterials vor Eintritt in die Entladungsstrecke so eingeregelt, daß die beim Aufschluß auftretende Temperaturerhöhung zu einer Endtemperatur im zulässigen Bereich führt.

Das erfindungsgemäße Verfahren wird vorzugsweise kontinuierlich durchgeführt. Es wird dazu laufend Rohstoff eingeführt, angemaischt und durch Zugabe von Lauge basisch gemacht.

Als Entladungsstrecke wird vorzugsweise ein vertikal angeordnetes Rohr verwandt, daß an seiner Innenseite paarweise angeordnete Elektroden aufweist. Die Elektroden bestehen beispielsweise aus Graphit. Das Substrat passiert das Entladungsrohr vorzugsweise von unten nach oben, wobei die Strömungsgeschwindigkeit 1 bis 5 l/min. beträgt.

Zur Verbesserung des Aufschlusses bis hin zu den freien Aminosäuren kann das angemaischte Ausgangsmaterial mehrfach durch die Entladungsstrecke geführt werden, beispielsweise bis zu viermal, wobei ein Umlaufverfahren eingesetzt werden kann. In der Regel ist aber unter den erfindungsgemäßen Verfahrensbedingungen ein Durchgang ausreichend.

Wird ein Umlaufverfahren verwandt, werden vorzugsweise etwa 10 bis 50 Gew.-%, insbesondere etwa 25 Gew.-%, des in Umlauf befindlichen behandelten Materials ausgeschleust und durch frisches angemaischtes Material ersetzt. Das ausgeschleuste Material wird zur Gewinnung der wäßrigen Hydrolysatphase weiterbehandelt, das mit frischem Material angereicherte Umlaufmaterial erneut durch die Entladungsstrecke geführt.

Die durch Abpressen der festen Rückstände und/oder Filtration erhaltene wäßrige Hydrolysatphase wird neutralisiert und zu festem Produkt aufbereitet, sofern sie nicht in den Anmaischbehälter zurückgeführt wird, um frisches Material anzumaischen. Im Anmaischbehälter werden vorzugsweise 10 bis 50 %, vorzugsweise etwa 25 % frisches Material mit 50 bis 90 %, vorzugsweise etwa 75 % wäßriger Hydrolysatphase vermischt.

Das erhaltene Hydrolysat wird auf an und für sich bekannte Weise in ein Pulver oder eine Paste überführt, welche beispielsweise als Grundstoff für die chemische, pharmazeutische oder Lebensmittelindustrie verwandt werden kann, aber auch als Futtermittel in der Landwirtschaft.

Die bisher angewandten Verfahren zur Gewinnung der Eiweißbausteine aus tierischen oder pflanzlichen Ausgangsmaterialien beruhten alle auf der Verwendung von Säuren oder Enzymen und/oder erhöhtem Druck und erhöhter Temperatur. Nach dem erfindungsgemäßen Verfahren erfolgt die Gewinnung von Eiweißbausteinen aus der Zellmasse mit Strom unter basischen Bedingungen. Bei Anwendung von Säuren und erhöhten Druck/Temeperatur- bedingungen sind Veränderungen der Eiweißbausteine bzw. Aminosäuren nicht zu vermeiden, so daß (häufig) ein weniger reines Produkt erhalten wird. Der Einsatz von Enzymen ist ausgesprochen teuer und nicht in jedem Falle selektiv. Erfindungsgemäß wird dagegen die Freisetzung der Aminosäuren ohne eine nachteilige chemische Veränderung erreicht. Zusammen mit den Eiweißbausteinen freigesetzte Aromaten, Stärken und Zucker können mit an und für sich bekannten Trenn- verfahren abgetrennt werden, das Aminosäuregemisch auf übliche Weise aufgetrennt werden.

Die im erfindungsgemäßen Verfahren gewonnenen Eiweiß- bausteine sind, je nach Aufschluß und Trennbedarf, rein verfügbar. Das erfindungsgemäß erhaltene Hydrolysat hat einen Trockensubstanzgehalt von bis zu 40 Gew.-% an Peptonen und/oder Aminosäuren. Bekannte Verfahren, die auf dem Zusatz von Fremdwasser beruhen, erreichen einen Trockensubstanzgehalt von 3 bis maximal 10 %. Das erfindungsgemäß erhaltene Produkt ist nach der Trocknung ein rieselfähiges und im wesentlichen geruchloses steriles Pulver bzw. Paste.

Die im erfindungsgemäßen Verfahren nicht aufgeschlossenen Zellteile, Keratine, Gewebestrukturen, Ligninstoffe und Restmaterialien können nach dem Abfördern/Abpressen weiter zu Futter- oder Düngemittel verarbeitet werden.

Die Erfindung bringt eine Reihe von Vorteilen mit sich, die die gesamte Verfahrensführung betreffen. Die Arbeitsweise im stark basischen Bereich bei erhöhter Temperatur erleichtert die Abtrennung von Fettbestandteilen bei der Aufarbeitung. Auf diese Weise kann auch stärker fetthaltiges Material in das Verfahren einbezogen werden und in aller Regel ohne eine Fettabtrennungsstufe in der Vorbereitung gearbeitet werden. Dies bringt eine deutliche Vereinfachung der Anlage mit sich und auch eine Ersparnis bei den Energiekosten, da die zur Fettabtrennung in der Vorbereitung benötigte Aufheizung fortfallen kann.

Des weiteren ergibt sich insgesamt eine kürzere Behandlungszeit in der Entladungsstrecke, da die Hydrolyse durch das basische Milieu gefördert wird. Die Base dient gleichzeitig als Elektrolyt, so daß in der Regel auf die Leitfähigkeit der Maische erhöhende Zusätze verzichtet werden kann. Die Verkürzung der Behandlungszeit erlaubt es, die Zahl der Durchgänge durch die Entladungsstrecke zu vermindern, so daß zumeist mit einer einmaligen Passage und entsprechend langer Nachbehandlung ein hinreichend aufgeschlossenes Produkt erhalten werden kann.

Des weiteren erhöht die erfindungsgemäß Arbeitsweise die Pumpfähigkeit der Maische, so daß mit höheren Konzentrationen an Ausgangsmaterial gearbeitet werden kann.

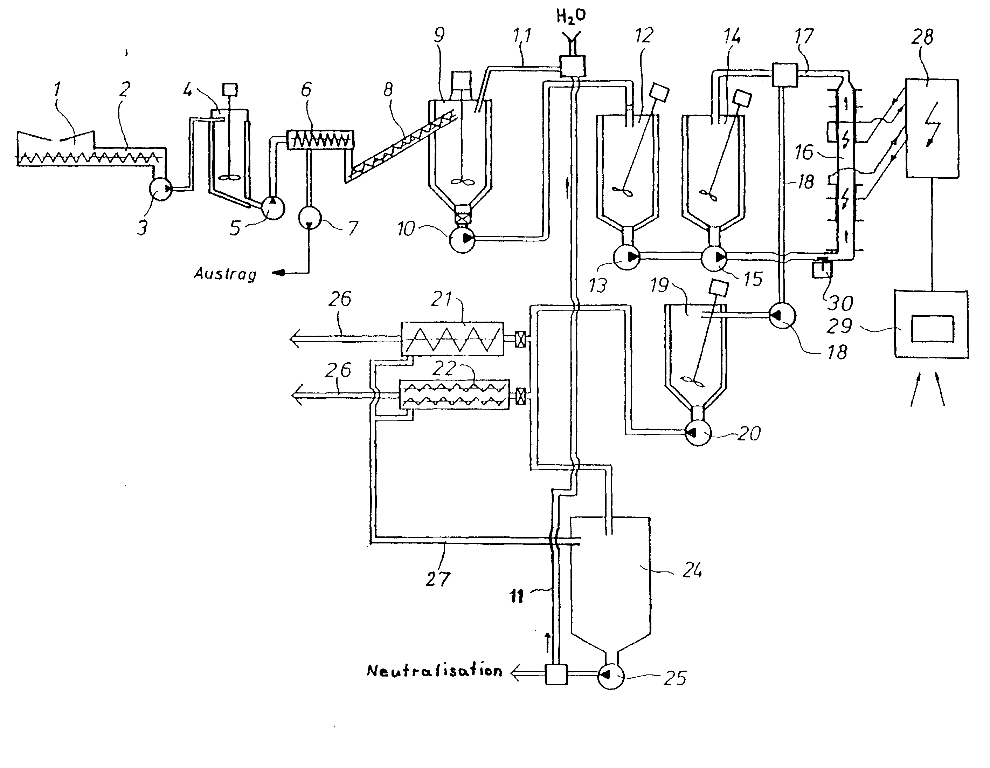

Die Erfindung wird mit Bezug auf die beiliegende Abbildung näher erläutert.

Das tierische oder pflanzliche Ausgangsmaterial wird zunächst einer Zerkleinerung zugeführt, beispielsweise einem üblichen Mahlwerk oder Wolf. Die Zerkleinerung sollte eine Partikelgröße von weniger als 6 bis 8 mm sicherstellen, wobei eine weitergehende Zerteilung des Ausgangsmaterials dem Verfahren förderlich ist, da die Pumpfähigkeit der Maische erhöht und der Aufschluß durch die Vergrößerung der Oberfläche erleichtert wird. Die Zerkleinerung findet im allgemeinen bei Umgebungstemperatur oder unter Kühlung statt, jedoch kann, insbesondere bei sehr fettreichen Materialien, auch bei 2 erwärmt werden, um eine Vorabtrennung des Fetts zu ermöglichen.

Die folgenden Verfahrensschritte sind insbesondere bei der Behandlung sehr fettreichen Materials unter Erwärmung hilfreich, sind aber für den Erfolg des erfindungsgemäßen Verfahrens nicht unbedingt erforderlich. Dabei wird das zerkleinerte Material bei 2 erwärmt werden.

Das Ausgangsmaterial wird mit einer Dickstoffpumpe 3 in ein Mischgefäß 4 mit Heizung und Rührwerk eingebracht und dort homogenisiert und auf die Verfahrenstemperatur aufgeheizt. Das derart homogenisierte Material wird mit einer Dickstoffpumpe 5 in eine Trennvorrichtung (Dekanter) 6 geführt, in der Fett und Wasser abgetrennt und mittels einer Pumpe 7 der weiteren Verarbeitung zugeführt werden. Anstelle eines Dekanters kann auch eine Doppelwellenpresse eingesetzt werden.

Das die Trennvorrichtung 6 verlassende Material wird über eine Schnecke 8 in den Vorlagetank 9 mit Rührwerk gefördert. In diesen Tank 9 kann über die Rücklaufleitung 11 wäßrige Hydrolysatphase zur Anmaischung des erhitzten und von Fett befreiten Materials eingebracht werden. Alternativ und bevorzugt wird aber mit Wasser angemaischt.

Aus dem Vorlagetank 9 gelangt das angemaischte Substrat über die Pumpe 10 in einen Dosierbehälter 12 zur Impulsstrecke, der ein Rührwerk und eine Dosiereinrichtung für Natronlauge aufweist. Vom Dosierbehälter 12 wird das angemaischte Material dann mit Hilfe einer Dosierpumpe 13 zur Impulsstrecke 16 mit den Entladungselementen geführt. Die Impulsstrecke kann mehrere Entladungselemente aufweisen oder es können mehrere Impulsstrecken parallel zu- oder hintereinander geschaltet sein.

Der in der Impulsstrecke eingeleitete Aufschlußprozeß erfolgt kontinuierlich. Vom Vorlagetank 9 wird über den Dosierbehälter 12 laufend Rohstoff zugeführt.

Die Impulsstrecke besteht vorzugsweise aus einem vertikal angeordneten Rohr, daß von unten nach oben durchströmt wird. Mit Hilfe der Dosierpumpe 13 und einer Zudosierpumpe 15 für Umlaufmaterial wird eine zuträgliche Strömungsgeschwindigkeit von 1 bis 5 l/min. sichergestellt, wobei der Durchsatz über einen Strömungswächter 30 überwacht und gesteuert werden kann, so daß die jeweils benötigte Verweildauer und die Temperatur eingehalten werden. Die Temperatur kann auch über eine Kühleinrichtung gesteuert werden.

Um einen vollen Aufschluß unter schonendsten Voraussetzungen bezüglich der angewandten Stromstärke in Abhängigkeit von der Impulszahl/Sekunde zu erreichen, kann das die Impulsstrecke durchströmende Material im Kreis gefahren werden. Es durchströmt die Impulsstrecke mehrfach, je nach Anforderung vorzugsweise bis zu viermal. Über den Anmaischbehälter 9 wird 25 % angemaischter Rohstoff zugefahren, 75 % des in der Impulsstrecke behandelten Materials werden in Umlauf gehalten, ausgeschleust werden 25 % der jeweils in Umlauf befindlichen Menge.

Das aus der Impulsstrecke 16 austretende Material gelangt in eine Leitung 17, die sich gabelt und einerseits in einen Umlaufbehälter 14 mit Rührwerk und Heizung bzw. Kühlung führt und andererseits über eine Ausschleusleitung und Pumpe 18 in den Nachreaktionsbehälter 19. Über die Ausschleuspumpe 18 werden vorzugsweise 10 bis 50 % des aufgeschlossenen Substrats und insbesondere etwa 25 % aus dem Umlauf genommen.

Das in den Umlaufbehälter 14 gelangte Material wird über eine Dosierpumpe 15 im Kreis zurück in die Impulsstrecke 16 geführt. Die Umlaufmenge selbst bestimmt sich aus dem von Dosierpumpen 13 und 15 in Abstimmung mit dem Strömungswächter 30 und einer Steuerung geförderten Material. Besonders bevorzugt sind 75 % Umlaufmaterial und 25 % zugemischtes angemaischtes Ausgangsmaterial.

Der Nachreaktionsbehälter 19 nimmt das in der Entladungsstrecke aufgeschlossene Material auf. Ein Rührer sorgt dafür, daß das Material in Bewegung gehalten werden kann, eine Heizeinrichtung, insbesondere eine solche, die über einen Heizmantel mittels Dampf eine geregelte Temperatursteuerung ermöglicht, sichert die für die Nachreaktion optimale Temperatur im Bereich von 70 bis 95°C. Temperatur und pH-Wert werden überwacht und, falls erforderlich, nachjustiert. Hierzu kann eine Dosiereinrichtung für Natronlauge vorhanden sein, die bei abgesunkenem pH-Wert diesen wieder in den Bereich von 10 bis 14 anhebt. Bei Einhaltung der vorgegebenen Verfahrensbedingungen hat sich gezeigt, daß sich der Aufschluß fortsetzt und sich rasch eine Phasentrennung in eine oben schwimmende Fettphase, eine Peptone und Aminosäuren enthaltende wäßrige Phase und eine untere Sedimentationsphase einstellt. Im allgemeinen bleibt in dieser Phase der pH-Wert, der sich in der Entladungsstrecke stark absenken kann, mehr oder weniger konstant.

Das aus dem Verfahren über die Ausschleuspumpe 18 ausgeschleuste Material im Nachreaktionsbehälter 19 gelangt über eine Pumpe 20 in eine Vortrennung, in der die Fettphase ab- und gelöstes und ungelöstes voneinander getrennt wird. Die Abtrennung der Fettphase erfolgt vorzugsweise mit einem Dekanter 21, die der festen Bestandteile in einem Vorfilter 22, aus denen jeweils Stoffe bei 26 ausgetragen werden und wäßriges Hydrolysat über die Leitung 27 in den Hydrolysatbehälter 24 gelangt. Der Hydrolysatbehälter 24 ist über eine Pumpe 25 und die Rücklaufleitung 11 mit dem Vorlagetank 9 verbunden, in dem Ausgangsmaterial mit dem Hydrolysat zur Pumpfähigkeit angemaischt werden kann.

Vom Hyddrolisatbehälter 24 gelangt das Hydrolisat dann in eine vorzugsweise beheizte Neutralisation, in der das Hydrolisat mit Schwefelsäure neutralisiert oder leicht angesäuert (pH 6,5) wird. An die Neutralisation schließt sich eine übliche Sterilfiltration an. Das steril filtrierte Material wird dann entwässert und der weiteren Verwendung zugeführt.

Das gewonnene Hydrolysat ist völlig steril und kann nach bekannten Verfahren weiter verarbeitet und insbesondere auf die einzelnen Aminosäuren hin aufgearbeitet werden. Das Hydrolysat selbst ist absolut steril und enthält nach dem Dekantieren, Abpressen und Filtrieren praktisch ausschließlich die jeweiligen Aminosäuren und/oder Peptone.

Die Hochspannung für die Impulsstrecke 16 wird mit Hilfe eines Generators 28 erzeugt. Der Strom wird über einen Zerhacker und Hochleistungskondensatoren in Mikrostößen auf die Entladungsstrecke gegeben. Steuerung und Kontrolle erfolgen über einen Oszillographen 29 für die Stromstärke und die Impulszahl/Zeiteinheit. Der Strom ist ein diskontinuierlicher Gleichstrom. Er kann ein Impulsstrom oder Entladungsstrom sein, wobei letzterer über Kondensatoren erzeugt wird. Kondensatoren einer Kapazität von 5 bis 25 µF und einer Impulshäufigkeit von 5 bis 30 Impulsen/s haben sich als besonders günstig erwiesen. Es wird vorzugsweise mit Hochspannung in einem Bereich von 2 bis 20 kV gearbeitet. Die Feldstärke ist abhängig vom Durchmesser der Impulsstrecke und mengenabhängig. Die Spannung kann bei Anlagen von 8 bis 10 t Stundenleistung bis zu 100 kV betragen. Spannung, Impulsfolge und Impulsdauer werden in Abhängigkeit vom eingesetzten Material so variiert, daß ein möglichst vollständiger Aufschluß der Proteinbestandteile des Substrats zu den einzelnen Aminosäuren erfolgt.

Das gewonnene Hydrolysat ist, insbesondere bei Sterilfiltration, frei von Mikroorganismen. Bei herkömmlichen Verfahren wurde diese Sterilität und dieses Arbeitsergebnis nur durch Arbeiten mit Zeit, Druck oder Säuren erreicht.

Die im Hydrolysat befindlichen Aminosäuren sind abtrennbar und gehen durch das genannte Verfahren keine neuen Bindungen ein.

### Beispiel 1

In der in der Abbildung gezeigten Anlage wurden 300 kg auf ≤ 8 mm gewolfte Schweinschwarte mit 15 1 25 %-iger Natronlauge angemeischt und gründlich vermischt. Es gab sich eingangs - pH-Wert von 12,2. Die Anfangstemperatur betrug 25°C, die durch Beheizen und die in der Impulsstrecke übertragene Wärme auf einen Wert ≥ 70 und < 80°C erhöht wurde.

Das Gemisch wurde mit einer Geschwindigkeit von 200 kg/h 2 Stunden durch die Impulsstrecke geführt. Die Spannung betrug 5,1 kV, die Stromstärke 3,0 A, die Kapazität 25 µS und der Widerstand 2,4 Ohm. Der pH-Wert blieb im wesentlichen konstant, das Gemisch selbst wurde zunehmend flüssiger und besser pumpfähig.

Nach Beendigung des Aufschlusses wurde das Gemisch bei laufendem Rührer einer Nachreaktion unterworfen, wobei die Temperatur von anfangs 75°C auf 92°C gesteigert wurde. Der pH-Wert wurde mit 50 l NAOH auf 12,85 gebracht, und das Gemisch zunächst 3 Stunden kräftig gerührt.

Anschließend blieb das Gemisch über Nacht stehen. Es trat auftrennung in eine untere wässrige und eine obere Fettphase auf, durch dekantieren wurden insgesamt 170 l wässrige Phase sowie 120 l Fettschlamm gewonnen. Letzterer wurde verworfen.

Die wässrige Phase wurde mit 15 %-iger H₂SO₄ auf einen pH-Wert von 6,5 gebracht und dann bei einer Temperatur von 80 bis 82°C einer Mikrofiltration mit einer Rate von anfangs 36 l/h unterworfen. Insgesamt wurden 175 l steril filtrierte Lösung gewonnen; es verblieben 35 1 Rückstand.

Durch Verdampfen des Restwassers wurde ein rieselfähiges Pulver gewonnen.

### Beispiel 2

230 kg schlachtfrische Schweineschwarten wurden auf ≤ 6 mm gewolft und mit 40 l Natronlauge auf ein pH-Wert von 11,3 gebracht. Nach Homogenisierung und Einstellung der Temperatur auf 75°C wurde das Gemisch in zwei aufeinanderfolgenden Durchgängen mit einer Geschwindigkeit von 90 bis 100 kg/h durch die Impulsstrecke geführt. Im ersten Durchgang wurde dazu die Temperatur von 20°C auf 75°C gesteigert, im zweiten Durchgang auf 80°C. Der pH-Wert blieb konstant bei 11,3. Die Impulsstrecke wurde mit einer Spannung von 3,4 kV und einer Stromstärke von 5 A (4,5 A im zweiten Durchgang) betrieben. Die Kapazität betrug 30 µF, der Widerstand 20 Ω (15 Ω im zweiten Durchgang).

Der Aufschuß war nach etwa 5 Stunden abgeschlossen, wonach mit 40 l Wasser nachgespült und erneut mit Natronlauge auf einen pH-Wert von 11,3 eingestellt wurde. Der Ansatz wurde bei 75°C über Nacht stehengelassen, danach auf 0,5 mm abgesiebt und dekantiert. Die wässrige Phase, insgesamt 200 l, wurde mit Schwefelsäure auf einen pH-Wert von 5,8 eingestellt und anschließend unter Druck mikrofiltriert, wie unter 1 beschrieben. Die Ausbeute an wässriger Lösung betrug 74 Gew.-%, einschließlich Natronlauge, und 26 % Fettwasser.

## Patentansprüche

1. Verfahren zur Erzeugung von Protein-Hydrolisaten aus animalischen und/oder vegetabilen Ausgangsmaterialien durch Zerkleinern der Ausgangsmaterialien, Anmaischen der zerkleinerten Ausgangsmaterialien zu einer pumpfähigen Masse, Aufschließen der angemaischten Ausgangsmaterialien in einer elektrischen Entladungsstrecke und Abtrennen der wäßrigen Phase des Aufschlusses von festen und nicht wassermischbaren Rückständen, **dadurch gekennzeichnet, daß** das Ausgangsmaterial bei einem pH-Wert zwischen 10,0 und 14,0 aufgeschlossen wird und das aufgeschlossene Ausgangsmaterial einer Nachreaktion über wenigstens 1 h bei einem pH-Wert von 10,0 bis 14,0 unterworfen wird, wobei die Temperatur wenigstens 70°C beträgt.

2. Verfahren nach Anspruch 1, **dadurch gekennzeichnet, daß** der Aufschluß mittels elektrischer Entladungen bei einer Temperatur von weniger als 95°C durchgeführt wird.

3. Verfahren nach Anspruch 2, **dadurch gekennzeichnet, daß** der Aufschluß bei einer auf 70 bis 85°C ansteigenden Temperatur durchgeführt wird.

4. Verfahren nach einem der vorstehenden Ansprüche, **dadurch gekennzeichnet, daß** der pH-Wert während des Aufschlusses im Bereich von 11,0 bis 13,5 liegt.

5. Verfahren nach einem der vorstehenden Ansprüche, **dadurch gekennzeichnet, daß** die Nachreaktion bei einer Temperatur von bis zu 95°C durchgeführt wird.

6. Verfahren nach Anspruch 5, **dadurch gekennzeichnet, daß** die Nachreaktion bei einer Temperatur von 75 auf 90°C durchgeführt wird.

7. Verfahren nach einem der vorstehenden Ansprüche, **dadurch gekennzeichnet, daß** die Nachreaktion bei einem pH-Wert von 11,0 bis 13,5 durchgeführt wird.

8. Verfahren nach einem der vorstehenden Ansprüche, **dadurch gekennzeichnet, daß** die Nachreaktionszeit 2 h bis 15 h beträgt.

9. Verfahren nach einem der vorstehenden Ansprüche, **dadurch gekennzeichnet, daß** als Base zur Einstellung des pH-Werts Natronlauge verwandt wird.

10. Verfahren nach einem der vorstehenden Ansprüche, **dadurch gekennzeichnet, daß** das der Aufschluß des angemaischten Ausgangsmaterials im Elektroimpulsverfahren bei einer Spannung von mehr als 2 kV mit einer Impulshäufigkeit von 5 bis 30 Impulsen/s durchgeführt wird, wobei die Fließgeschwindigkeit in der Impulsstrecke 1 bis 5 l/min. beträgt.

11. Verfahren nach einem der vorstehenden Ansprüche, **dadurch gekennzeichnet, daß** das Ausgangsmaterial mit Wasser angemaischt wird.

12. Verfahren nach einem der vorstehenden Ansprüche, **dadurch gekennzeichnet, daß** die abgetrennte wäßrige Phase des Aufschlusses mit einer Säure auf einen pH-Wert von 5,0 bis 7,0 gebracht und anschließend bei einer Temperatur von 50 bis 90°C steril filtriert wird.

13. Verfahren nach einem der vorstehenden Ansprüche, **dadurch gekennzeichnet, daß** die abgetrennte wäßrige Phase des Aufschlusses im Sprühturm getrocknet wird.

## Claims

1. Method of producing protein hydrolysates from animal and/or vegetable starting materials by comminuting the starting materials, mashing to the comminuted starting materials to form a pumpable composition, hydrolysing the mashed starting materials in an electrical discharge space and separating the aqueous phase of the hydrolysate from the solid phase and residues which are immiscible with water, **characterised in that** the starting material is hydrolysed at a pH value of between 10.0 and 14.0 and the hydrolysed starting material is subjected to a subsequent reaction for at least 1 h at a pH value of 10.0 to 14.0, the temperature being at least 70°C.

2. Method as claimed in Claim 1, **characterised in that** the hydrolysis is performed by means of electrical discharges at a temperature of less than 95°C.

3. Method as claimed in Claim 2, **characterised in that** the hydrolysis is performed at a temperature rising to 70 to 85°C.

4. Method as claimed in one of the preceding claims, **characterised in that** the pH value during the hydrolysis is in the range of 11.0 to 13.5.

5. Method as claimed in one of the preceding claims, **characterised in that** the subsequent reaction is performed at a temperature of up to 95°C.

6. Method as claimed in Claim 5, **characterised in that** the subsequent reaction is performed at a temperature of 75 to 90°C.

7. Method as claimed in one of the preceding claims, **characterised in that** the subsequent reaction is performed at a pH value of 11.0 to 13.5.

8. Method as claimed in one of the preceding claims, **characterised in that** the subsequent reaction time is 2 h to 15 h.

9. Method as claimed in one of the preceding claims, **characterised in that** caustic soda solution is used as a base to adjust the pH value.

10. Method as claimed in one of the preceding claims, **characterised in that** the hydrolysis of the mashed starting material in an electric pulse process is effected at a voltage of more than 2 kV with a pulse frequency of 5 to 30 pulses/s, the flow velocity in the pulse region being 1 to 5 l/min.

11. Method as claimed in one of the preceding claims, **characterised in that** the starting material is mashed with water.

12. Method as claimed in one of the preceding claims, **characterised in that** the separated aqueous phase of the hydrolysate is brought with an acid to a pH value of 5.0 to 7.0 and is subsequently sterile filtered at a temperature of 50 to 90°C.

13. Method as claimed in one of the preceding claims, **characterised in that** the separated aqueous phase of the hydrolysate is dried in a spray tower.

## Revendications

1. Procédé pour la préparation d'hydrolysats de protéine à partir de matières premières animales et/ou végétales par fractionnement des matières premières, trempage des matières premières fractionnées pour former une masse pompable, désagrégation des matières premières trempées dans un espace de décharge électrique et séparation de la phase aqueuse du produit de désagrégation des résidus solides et non miscibles à l'eau, **caractérisé en ce que** la matière première est désagrégée à un pH entre 10,0 et 14,0 et la matière première désagrégée est soumise à une réaction subséquente pendant au moins 1 h à un pH de 10,0 à 14,0, tandis que la température est d'au moins 70°C.

2. Procédé selon la revendication 1, **caractérisé en ce que** la désagrégation est effectuée au moyen de décharges électriques à une température inférieure à 95°C.

3. Procédé selon la revendication 2, **caractérisé en ce que** la désagrégation est effectuée à une température s'élevant de 70 à 85°C.

4. Procédé selon l'une des revendications précédentes, **caractérisé en ce que** le pH pendant la désagrégation se situe dans l'intervalle de 11,0 à 13,5.

5. Procédé selon l'une des revendications précédentes, **caractérisé en ce que** la réaction subséquente est effectuée à une température allant jusqu'à 95°C.

6. Procédé selon la revendication 5, **caractérisé en ce que** la réaction subséquente est effectuée à une température de 75 à 90°C.

7. Procédé selon l'une des revendications précédentes, **caractérisé en ce que** la réaction subséquente est effectuée à un pH de 11,0 à 13,5.

8. Procédé selon l'une des revendications précédentes, **caractérisé en ce que** la durée de la réaction subséquente est de 2 h à 15 h.

9. Procédé selon l'une des revendications précédentes, **caractérisé en ce qu'**on utilise de la lessive de soude comme base pour l'ajustement du pH.

10. Procédé selon l'une des revendications précédentes, **caractérisé en ce que** la désagrégation de la matière première trempée dans l'opération d'impulsion électrique est effectuée à une tension supérieure à 2 kV avec une fréquence d'impulsions de 5 à 30 impulsions/s, tandis que la vitesse d'écoulement dans l'espace d'impulsions est de 1 à 5 l/min.

11. Procédé selon l'une des revendications précédentes, **caractérisé en ce que** la matière première est trempée avec de l'eau.

12. Procédé selon l'une des revendications précédentes, **caractérisé en ce que** la phase aqueuse séparée du produit de désagrégation est amenée avec un acide à un pH de 5,0 à 7,0 et ensuite filtrée à une température de 50 à 90°C dans des conditions stériles.

13. Procédé selon l'une des revendications précédentes, **caractérisé en ce que** la phase aqueuse séparée du produit de trempage est séchée dans une tour à pulvérisation.
